Europäisches Patentamt

European Patent Office

Office européen des brevets

(19)

(11) Numéro de publication : **0 180 532 B1**

(12) **FASCICULE DE BREVET EUROPÉEN**

(45) Date de publication du fascicule du brevet :
15.03.89

(51) Int. Cl.⁴ : **A 61 B 17/58**

(21) Numéro de dépôt : **85460016.0**

(22) Date de dépôt : **16.10.85**

(54) **Coapteur pour fractures du col du fémur et similaires.**

(30) Priorité : **24.10.84 FR 8416512**

(43) Date de publication de la demande :
**07.05.86 Bulletin 86/19**

(45) Mention de la délivrance du brevet :
**15.03.89 Bulletin 89/11**

(84) Etats contractants désignés :
**AT BE CH DE GB IT LI LU NL SE**

(56) Documents cités :
**DE–C– 103 873**
**DE–C– 745 873**
**DE–C– 913 838**
**FR–A– 2 068 103**
**FR–A– 2 342 710**
**FR–A– 2 531 153**
**US–A– 2 077 804**
**US–A– 2 121 193**
**US–A– 2 327 434**
**US–A– 2 877 818**
**US–A– 3 029 811**
**US–A– 4 409 974**

(73) Titulaire : **UNIVERSITE DE RENNES I**
**2, rue du Thabor**
**F-35000 Rennes (FR)**

(72) Inventeur : **Chagneau, Francis**
**15, allée d'Hennebont**
**F-35700 Rennes (FR)**
Inventeur : **Levasseur, Michel**
**40, rue de la Coulée**
**F-35510 Cesson-Sevigne (FR)**

(74) Mandataire : **Martin, Jean-Jacques et al**
**Cabinet REGIMBEAU Corre, Martin, Schrimpf, War-**
**coin, Ahner 11, rue Franz Heller**
**F-35700 Rennes (FR)**

## Description

La présente invention concerne un dispositif d'ancrage des fractures du col du fémur ou de fractures similaires, appelé coapteur.

Dans les techniques d'ostéosynthèse du col du fémur connues, on utilise généralement un dispositif d'ancrage en forme de potence. Celle-ci comprend une plaque allongée, galbée, destiné à être fixée au moyen de vis à la partie supérieure de la diaphyse fémorale, et un fût qui forme un angle obtus avec la plaque et qui est destiné à être enfoncé dans l'épiphyse fémorale à travers la fracture du col.

Certains dispositifs, dans lesquels le fût a simplement la forme d'un clou, n'assurent pas la compression des deux parties disjointes l'une contre l'autre.

Dans d'autres dispositifs, le fût comprend une broche filetée qui peut être vissée dans l'épiphyse fracturée de manière à réaliser une certaine compression des parties disjointes l'une contre l'autre. Malheureusement, l'expérience a montré que la pose de cette broche se révèle problématique en raison du risque d'une détérioration de l'os spongieux par les filets de la broche. Un dispositif de ce genre est connu par exemple par le document FR-A-2 342 710.

L'invention résout ces problèmes en proposant un coapteur, c'est-à-dire un dispositif d'ancrage des fractures du col du fémur, qui permette une compression efficace et contrôlée des parties disjointes, tout en étant simple à poser, résistant, et d'un prix de revient faible.

A cet effet, le dispositif objet de la présente invention comprend, comme les dispositifs connus, une plaque allongée destinée à être fixée à la diaphyse fémorale et un fût sensiblement cylindrique destiné à être logé dans un alésage traversant le col du fémur fracturé, le fût et la plaque étant reliés par leurs extrémités en formant un angle obtus. Conformément à l'invention, le fût est pourvu d'une ailette à bord coupant qui est située à l'extrémité du fût opposée à la plaque et est disposée sensiblement transversalement par rapport à l'axe du fût, cette ailette étant montée à pivotement autour d'un axe qui est légèrement décalé angulairement et/ou latéralement par rapport à l'axe du fût.

Ainsi, en mettant l'ailette en correspondance avec l'extrémité du fût, il est possible de mettre en place le dispositif ; en faisant tourner l'ailette d'un angle maximal de 180°, on la décale par rapport à l'extrémité du fût en la faisant pénétrer dans le tissu osseux avoisinant.

L'ailette est avantageusement mobile en translation en direction de la plaque ; il est ainsi possible de comprimer, au moyen de l'ailette, les parties disjointes, de manière contrôlée et réglable. Dans une forme de réalisation préférentielle du dispositif, l'ailette est portée par une tige montée à rotation et à coulissement longitudinal dans le fût, et dont l'extrémité opposée est filetée ; le déplacement de l'ailette en translation est alors réalisé par vissage d'un écrou sur cette extrémité filetée, l'écrou prenant appui contre l'extrémité correspondante du fût.

La forme de l'ailette est avantageusement circulaire, de diamètre voisin de celui du fût.

L'ailette peut également avoir une forme de pale. En donnant à celle-ci une forme hélicoïdale, il est possible d'obtenir un ancrage amélioré du dispositif dans le tissu osseux car, au cours de sa rotation, l'ailette provoque le déplacement de la tête du fémur contre le trochanter, réalisant la compression souhaitée des parties disjointes.

Le fût est muni de préférence de nervures longinales périphériques qui assurent son immobilisation en rotation au cours de l'opération. Il présente également, éventuellement, une rainure longitudinale apte à assurer son guidage par une broche lors de sa mise en place.

D'autres particularités et avantages de l'invention apparaîtront de la description et des dessins annexés qui présentent des modes de réalisation préférentiels de l'invention.

La figure 1 représente, en vue de face coupée, une première forme de réalisation du dispositif selon l'invention mis en place dans une épiphyse fémorale fracturée, avant ancrage ;

La figure 2 est une section d'une partie du dispositif, coupé par le plan II de la figure 1 ;

La figure 3 est une vue analogue à la figure 1 montrant le dispositif après ancrage mais avant compression ;

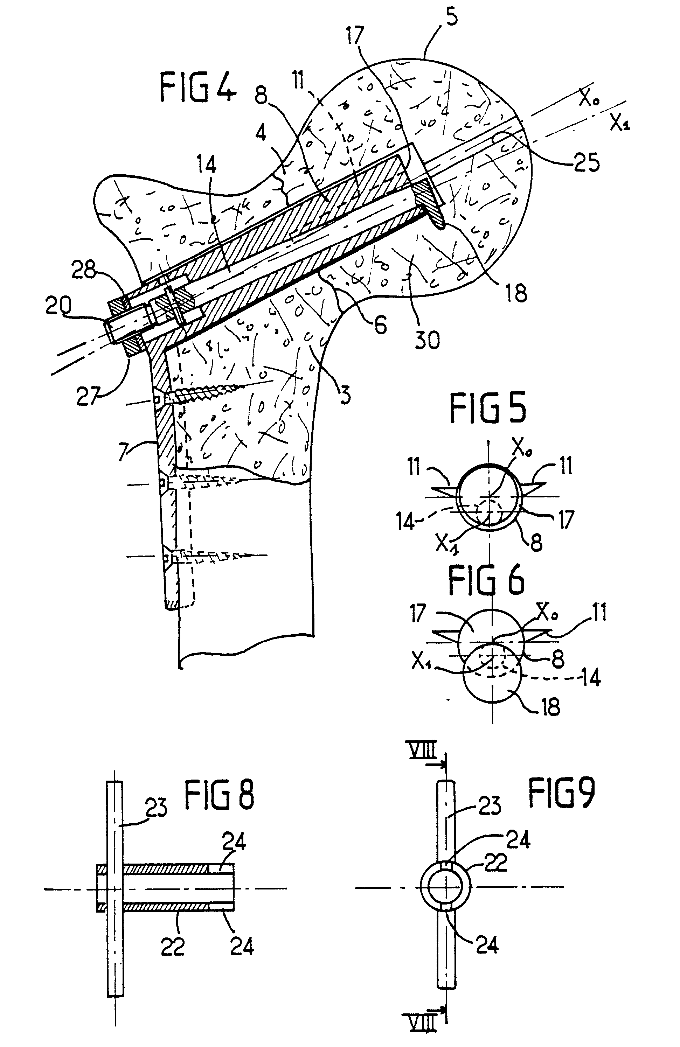

La figure 4 est une vue analogue aux figures 1 et 3 montrant le dispositif après compression ;

Les figures 5 et 6 sont des vues en bout du dispositif, vues selon les plans V et VI des figures 1 et 3 respectivement ;

La figure 7 est une vue de côté d'un outil d'ancrage apte à actionner le dispositif des figures 1 à 6 ;

La figure 8 est une vue de face de cet outil coupé par le plan VIII-VIII de la figure 7 ;

La figure 9 représente, en vue de face coupée, une seconde forme de réalisation d'un dispositif conforme à l'invention, après mise en place dans une épiphyse fémorale (non représentée) ;

Les figures 10 et 11 sont des vues partielles analogues à la figure 9 montrant le dispositif respectivement après ancrage et après compression ;

La figure 12 est une vue en bout du dispositif de la figure 9, vu suivant la flèche XII ;

La figure 13 est une vue en perspective de l'extrémité d'une troisième forme de réalisation d'un dispositif conforme à l'invention.

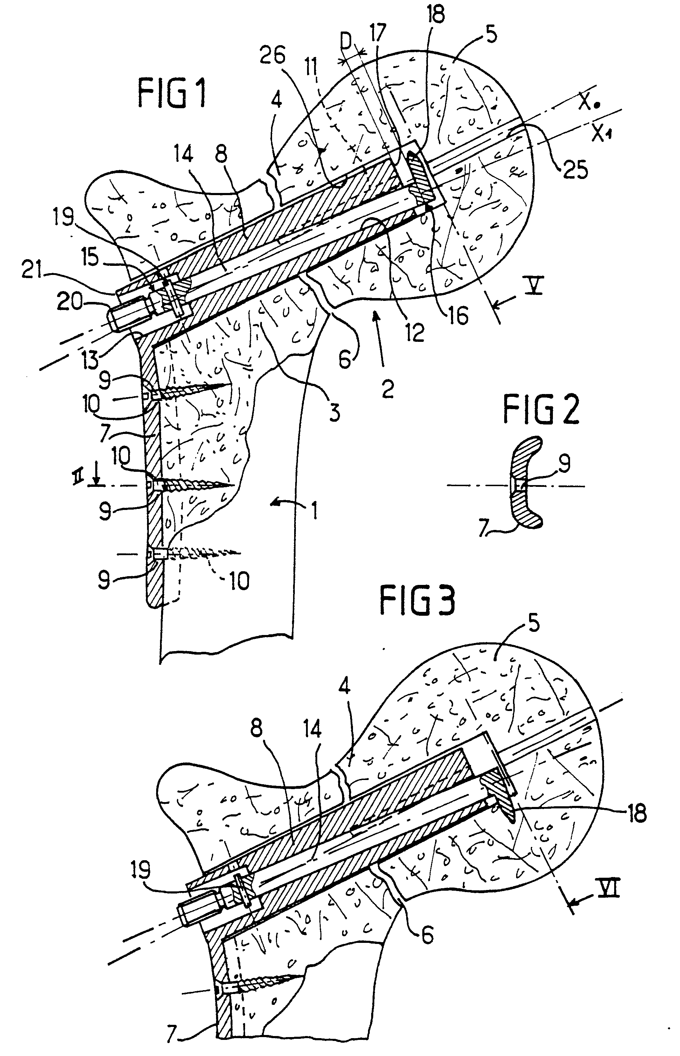

La figure 1 représente l'extrémité supérieure d'un fémur, qui comprend la diaphyse 1 et l'épiphyse 2.

Cette dernière comprend le trochanter 3, le col 4 et la tête 5. Les parties 3 et 5 sont disjointes au niveau du col par une fracture 6 que l'on se propose de ressouder par ostéosynthèse.

Le dispositif d'ostéosynthèse a la forme d'une potence qui comprend une plaque allongée 7 et un fût cylindrique 8 formant parties intégrantes l'une de l'autre. Le dispositif est en métal, par exemple en acier inoxydable ou en matière plastique à haute résistance.

La plaque 7 a une section incurvée, comme on le voit sur la figure 2, adaptée pour épouser au moins approximativement la partie supérieure latérale externe de l'épiphyse fémorale. Elle comporte plusieurs trous, par exemple trois trous 9, pour le passage de vis de fixation 10.

Le fût 8 forme avec la plaque 7 un angle obtus, de l'ordre de 135° par exemple. Sa paroi est pourvue d'une paire de nervures longitudinales 11, de préférence à bord effilé, qui s'étendent sur une partie au moins de sa longueur.

Le fût 8 est percé par un alésage cylindrique 12 dont l'axe $X_1$ est décalé angulairement par rapport à l'axe $X_0$ du fût. Ce décalage angulaire, de quelques degrés, est tel que le centre de l'alésage 12 coïncide approximativement avec le centre du fût 8 à son extrémité située du côté de la plaque 7 (sur la gauche des figures), et soit sensiblement déporté latéralement — par exemple vers le bas — par rapport au centre du fût à son autre extrémité (sur la droite des figures). A l'extrémité située du côté de la plaque 7, l'alésage 12 débouche dans un alésage 13 de plus grand diamètre.

Dans l'alésage 12 est montée une tige 14, de diamètre correspondant dont l'une des extrémités 15 fait saillie dans l'alésage 13 et l'autre extrémité 16 fait saillie au-delà de la face frontale 17 du fût 8.

L'extrémité 16 porte une ailette en forme de disque 18 à bord coupant dont le diamètre est voisin de celui de la face 17. Le disque 18 forme partie intégrante de la tige 14 ou, au contraire, est rapporté sur celle-ci par tout moyen approprié tel que soudage, rivetage ou vissage. Le centre du disque 18 est excentré par rapport au centre de la face 17 d'une valeur qui correspond au décentrage des axes $X_1$ et $X_0$ en cet endroit.

L'extrémité 15 est traversée par une goupille 19 et présente une partie filetée 20, de même diamètre que la tige 14, qui dépasse du bord extérieur 21 du fût 8.

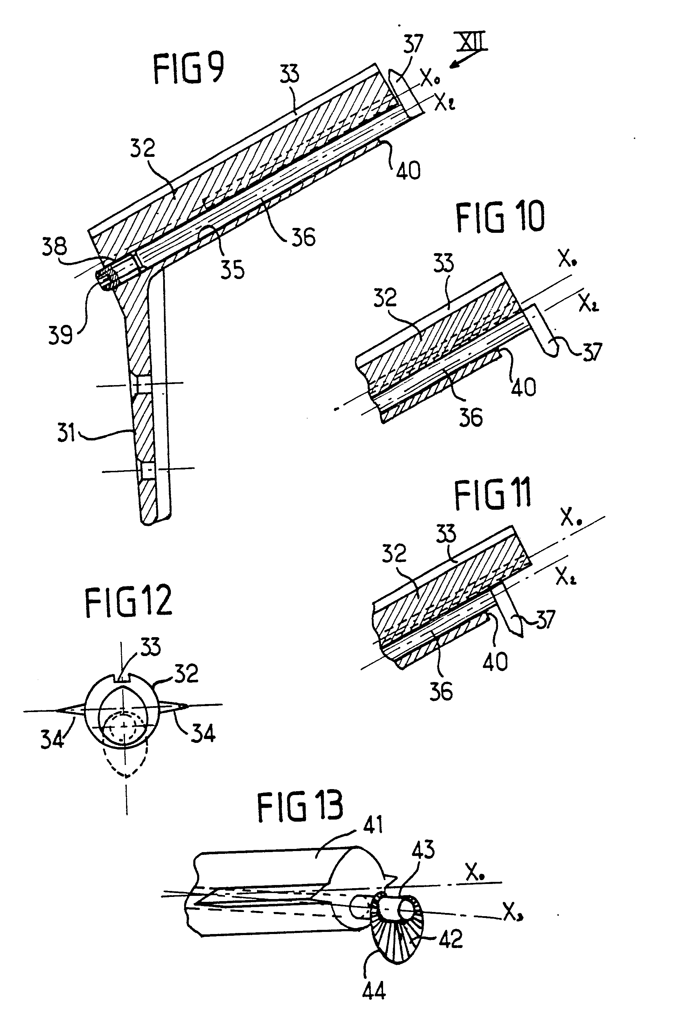

L'outil d'ancrage, représenté aux figures 8 et 9, est constitué par un manchon cylindrique 22 traversé par une tige de manœuvre 23. Le diamètre extérieur du manchon 22 est légèrement inférieur à celui de l'alésage 13. Son diamètre intérieur est légèrement supérieur à celui de la tige 14 et de la partie filetée 20. Le manchon est fraisé radialement de manière à présenter deux lumières longitudinales 24. Celles-ci sont situées à l'extrémité opposée à celle qui porte la tige de manœuvre 23.

Nous allons maintenant décrire la manière dont on utilise ce dispositif dans une opération d'ostéosynthèse du col du fémur 4.

On introduit, de manière connue, une broche à travers le trochanter, le col et la tête du fémur et on perce un alésage 26 de diamètre égal ou légèrement supérieur à celui du fût 8, coaxialement à la broche, en utilisant cette dernière comme organe de guidage. Il s'agit d'un alésage borgne, dont la longueur est légèrement supérieure à celle du fût 8.

Le trou percé par la broche est désigné par la référence 25 sur les dessins.

Après perçage, on retire la broche et on introduit le fût 8 dans l'alésage 26 jusqu'à ce que la plaque 7 s'applique contre le bord latéral externe de la partie supérieure de la diaphyse fémorale. Durant cette introduction, l'ailette discoïde 18 se trouve exactement en vis-à-vis de la partie frontale 17 du fût 8, contre celle-ci.

Les nervures latérales 11 du fût se logent dans le tissu osseux avoisinant au cours de l'enfoncement du fût dans le trou 26.

Ensuite, on fixe la plaque 7 à la diaphyse fémorale au moyen des vis 10, et on pousse sur la tige 14 pour décoller l'ailette 18 de la face frontale 17 d'une certaine distance D ; cette distance est de quelques millimètres, par exemple de 5 mm. Puis on engage le manchon 22 de l'outil d'ancrage sur l'extrémité 15 de la tige 14 de telle sorte que les lumières 24 viennent entourer les extrémités en saillie de la goupille 19. Ensuite, le chirurgien fait tourner cet outil d'un demi-tour au moyen de la poignée 23, ce qui entraîne la rotation correspondante, à la manière d'un loquet, du disque coupant 18. Ce dernier pénètre dans l'os spongieux qui l'entoure. Les nervures 11 empêchent que le fût ne tourne lui aussi durant la rotation du disque. L'ancrage est très efficace et le tissu osseux reste sain de part et d'autre du disque. Cette situation est illustrée à la figure 3.

Ensuite, on retire l'outil d'ancrage et on visse un écrou 27, éventuellement par l'intermédiaire d'une rondelle de blocage 28, sur la partie filetée 20 de la tige 14. Durant ce vissage, l'écrou 27 — ou éventuellement la rondelle 28 — prend appui contre la face 21 du fût 8 et on réalise une traction sur la tige 14. Celle-ci coulisse en direction de la plaque 7 ; l'ailette discoïde 18 comprime le tissu osseux 30 situé en vis-à-vis, et provoque le déplacement, puis le contact intime, de la tête de fémur 5 avec le trochanter 3 au niveau de la fracture. Ce mouvement longitudinal de la tige 14 et de l'ailette 18 est possible du fait de l'existence du jeu initial D entre cette ailette et la face frontale 17 du fût 8. L'amplitude de ce mouvement peut être réglé par le chirurgien en fonction du type de la fracture et de la qualité du tissu osseux.

En cas de bris du niveau de l'ailette, ou d'incidents divers, un démontage est toujours possible. De plus, on peut utiliser un nouveau dispositif d'ostéosynthèse, de longueur de fût un peu plus courte car le tissu osseux de la tête du fémur n'a pas été détérioré contrairement à ce qui se passe avec les systèmes à vis habituels (qui jouent alors le rôle d'emporte-pièce).

L'appui précoce après opération devrait être possible en raison d'une part de la grande résistance à la flexion de la potence constituée par la plaque métallique et le fût (dont le diamètre est relativement grand), et d'autre part de la qualité de l'ancrage.

On notera qu'il est possible d'intervenir sur la compression en agissant sur la vis, même en cours de service. Par ailleurs, le dispositif est démontable ; il suffit pour cela de reprendre les opérations précédentes dans l'ordre inverse.

Le dispositif, représenté aux figures 9 à 12, comprend une plaque incurvée 31 et un fût 32 d'axe $X_0$ formant un angle obtus avec ladite plaquette.

Sur le dessus du fût 32 est ménagée une rainure longitudinale 33 ; le fût 32 est pourvu de nervures latérales 34 similaires aux nervures 11 du dispositif décrit précédemment.

Le fût 32 est percé d'un alésage 35 dont l'axe $X_2$ est parallèle mais décalé vers le bas par rapport à l'axe $X_0$. Dans cet alésage est logée une tige 36 qui est pourvue à son extrémité interne (destinée à être logée dans l'épiphyse fémorale) d'une ailette coupante en forme de pale 37. A son extrémité externe la tige 36 possède une partie filetée 38 dont la face frontale présente une empreinte hexagonale 39.

Le bord inférieur 40 de l'extrémité interne de l'alésage 35 est échancré, la matière ayant été enlevée en cet endroit.

Ce dispositif est mis en place de manière similaire à celle décrite précédemment pour la première forme de réalisation. Toutefois, dans ce cas, la rainure 33 permet un guidage du fût 32 au moyen d'une broche préalablement enfoncée dans l'épiphyse fémorale (non représentée). La rotation de la pale 37 est commandée au moyen d'une clé alène qui est introduite dans l'empreinte 39 ; après un demi-tour la pale 37 fait saillie vers le bas en raison du décalage des axes $X_0$ et $X_2$ (figures 10 et 12) ; le déplacement axial de la tige 36 est obtenu, comme dans la forme de réalisation précédente, au moyen d'un écrou que l'on visse sur la partie filetée 38. Ce déplacement est autorisé par la présence de l'échancrure 40 qui ne contrarie pas le mouvement longitudinal de la pale 37 (figure 11).

La figure 13 représente (partiellement) une troisième forme de réalisation d'un dispositif conforme à l'invention. Ce dispositif comprend un fût 41, d'axe $X_0$, analogue à ceux précédemment décrits. Il comporte une ailette 42 portée par un arbre 43 guidé en rotation dans le fût 41. L'axe $X_3$ de l'arbre 43 est décalé soit angulairement, soit latéralement, soit à la fois angulairement et latéralement, par rapport à l'axe $X_0$. La pale 32, et notamment son bord coupant 44, ont une forme hélicoïdale.

Après mise en place de ce dispositif dans l'épiphyse fémorale, on fait tourner la tige 43 d'un demi-tour ; le bord coupant mord alors dans le tissu osseux avoisinant et provoque, en raison de sa forme en hélice, le déplacement de la tête du fémur en direction du col. On réalise ainsi la compression souhaitée sans qu'un déplacement longitudinal de la tige 43 ne soit nécessaire.

Il va de soi que l'invention n'est pas limitée aux formes de réalisation préférentielle que l'on vient de décrire, à simple titre d'exemples ; elle en embrasse au contraire toutes les variantes. C'est ainsi qu'il serait possible de réaliser un guidage de la tige portant l'ailette d'ancrage dans son alésage, qui soit agencé de telle manière que la rotation de cette tige entraîne automatiquement son déplacement axial ; un tel résultat peut être obtenu simplement en donnant à une partie de la tige la forme d'une vis sans fin à pas élevé et à une partie du fût la forme d'un écrou complémentaire.

Il est possible de donner au fût une forme étagée. Le fût est alors formé de deux tronçons de diamètres différents ; le diamètre du tronçon interne (du côté de l'ailette) est légèrement plus petit que le diamètre du tronçon externe (du côté de la plaque allongée). Cette disposition évite d'avoir à percer un alésage trop grand dans la tête du fémur, et, éventuellement dans le col au voisinage de la fracture, le tissu osseux étant généralement fragile dans ces régions.

Il va de soi que certaines caractéristiques additionnelles décrites en référence avec une forme de réalisation pourraient, sans sortir du cadre de l'invention, être adaptées à une autre forme de réalisation. C'est ainsi par exemple que la rainure de guidage 33 de la deuxième forme de réalisation pourrait également être prévue sur la première.

Il est possible de prévoir une série de coapteurs conforme à l'invention qui présentent des formes et des dimensions différentes de celles qui ont été représentées, afin de pouvoir être adaptées à des fractures du col du fémur de type différent ou à d'autres types de fractures (par exemple à des fractures de la tête de l'humérus). Ainsi, dans certaines applications, il est avantageux de raccourcir considérablement, voire de supprimer la plaque 7 ; dans d'autres applications, il est avantageux au contraire de l'allonger ou même de la prolonger au-delà de l'axe du fût ; le coapteur présenterait alors la forme approximative d'un T (au lieu de la forme de L décrite).

## Revendications

1. Coapteur pour fractures du col du fémur, ou similaires, qui comprend une plaque allongée destinée à être fixée à la diaphyse fémorale et un fût sensiblement cylindrique destiné à être logé dans un alésage traversant le col du fémur fracturé, ledit fût étant relié par l'une de ses extrémités à l'extrémité supérieure de la plaque de manière à former avec elle un angle obtus, caractérisé en ce qu'il comporte une ailette à bord coupant (18 ; 37 ; 42) située à l'extrémité du fût (8 ; 32 ; 41) opposée à la plaque allongée (7 ; 31) et disposée sensiblement transversalement à l'axe ($X_0$) du fût, cette ailette (18 ; 37 ; 42) étant montée à pivotement autour d'un axe ($X_1$ ; $X_2$ ; $X_3$) qui est légèrement décalé angulairement et/ou latéralement par rapport à l'axe ($X_0$) du fût.

2. Coapteur selon la revendication 1 caractérisé en ce que l'ailette (18 ; 37) est mobile en translation selon son axe de pivotement ($X_1$ ; $X_2$).

3. Coapteur selon la revendication 2, caractérisé en ce que l'ailette (18 ; 37) est portée par une tige (14 ; 36) présentant une partie d'extrémité filetée (20 ; 38) apte à coopérer avec un écrou de serrage (27).

4. Coapteur selon l'une des revendications 1 à 3, caractérisé en ce que l'ailette (18 ; 37) est portée par une tige (14 ; 36) dont l'extrémité (15 ; 38) est munie d'un moyen (19 ; 39) de commande de son pivotement.

5. Coapteur selon l'une des revendications 1 à 4, caractérisé en ce que l'ailette (18) a la forme d'un disque dont le diamètre est voisin de celui du fût (8).

6. Coapteur selon l'une des revendications 1 à 5, caractérisé en ce que l'ailette (37 ; 42) a la forme d'une pale.

7. Coapteur selon la revendication 6, caractérisé en ce que l'ailette (42) a la forme d'une pale hélicoïdale.

8. Coapteur selon l'une des revendications 1 à 7, caractérisé en ce que le fût est muni de nervures longitudinales (11 ; 34) aptes à assurer son immobilisation en rotation.

9. Coapteur selon l'une des revendications 1 à 8, caractérisé en ce que fût est muni d'une rainure longitudinale (33) apte à recevoir une broche de guidage.

**Claims**

1. Coaptor for fractured femural necks or the like, which comprises an elongate plate intended to be fixed to the femural diaphysis and a substantially cylindrical shaft intended to be received in a bore passing through the neck of the fractured femur, the said shaft being connected by one of its ends to the upper end of the plate so as to form with it an obtuse angle, characterized in that it has a lug with a cutting edge (18 ; 37 ; 42) located at the end of the shaft (8 ; 32 ; 41) opposite the elongate plate (7 ; 31) and arranged substantially transversely to the axis $(X_0)$ of the shaft, this lug (18 ; 37 ; 42) being mounted pivotally about an axis $(X_1 ; X_2 ; X_3)$ which is slightly offset angularly and/or laterally with respect to the axis $(X_0)$ of the shaft.

2. Coaptor according to Claim 1, characterized in that the lug (18 ; 37) is capable of translational movement along its axis of pivoting $(X_1 ; X_2)$.

3. Coaptor according to Claim 2, characterized in that the lug (18 ; 37) is carried by a rod (14 ; 36) which has a threaded end part (20 ; 38) suitable for cooperating with and adjusting nut (27).

4. Coaptor according to one of Claims 1 to 3, characterized in that the lug (18 ; 37) is carried by a rod (14 ; 36) whereof the end (15 ; 38) is provided with a means (19 ; 39) for controlling its pivoting.

5. Coaptor according to one of claims 1 to 4, characterized in that the lug (18) has the shape of a disc whereof the diameter is close to that of the shaft (8).

6. Coaptor according to one of Claims 1 to 5, characterized in that the lug (37 ; 42) has the shape of a blade.

7. Coaptor according to Claim 6, characterized in that the lug (42) has the shape of a helical blade.

8. Coaptor according to one of Claims 1 to 7, characterized in that the shaft is provided with longitudinal ribs (11 ; 34) suitable for assuring its rotational immobilisation.

9. Coaptor according to one of Claims 1 to 8, characterized in that the shaft is provided with a longitudinal (33) suitable for receiving a guide pin.

**Patentansprüche**

1. Koaptor für Oberschenkelhalsbrüche oder dergleichen, enthaltend eine längliche Platte zur Befestigung an der Oberschenkeldiaphyse und einen im wesentlichen zylindrischen Schaft, der zur Anbringung in einer Bohrung bestimmt ist, die den Hals des gebrochenen Oberschenkels durchdringt, welcher Schaft an einem seiner Enden mit dem oberen Ende der Platte derart verbunden ist, daß er mit dieser einen stumpfen Winkel bildet, dadurch gekennzeichnet, daß er einen Flügel mit Schneidkante (18 ; 37 ; 42) aufweist, der an dem Ende des Schaftes (8 ; 32 ; 41) angeordnet ist, das der länglichen Platte (7 ; 31) abgewandt ist, und der im wesentlichen quer zur Achse $(X_0)$ des Schaftes angeordnet ist, wobei dieser Flügel (18 ; 37 ; 42) schwenkbar um eine Achse $(X_1 ; X_2 ; X_3)$ montiert ist, die gegenüber der Achse $(X_0)$ des Schaftes im Winkel und/oder in Querrichtung leicht versetzt ist.

2. Koaptor nach Anspruch 1, dadurch gekennzeichnet, daß der Flügel (18 ; 37) translatorisch gemäß seiner Schwenkachse $(X_1 ; X_2)$ beweglich ist.

3. Koaptor nach Anspruch 2, dadurch gekennzeichnet, daß der Flügel (18 ; 37) von einem Stab (14 ; 36) gehalten ist, der einen mit einem Gewinde versehenen Endabschnitt (20 ; 38) aufweist, der zum Zusammenwirken mit einer Feststellmutter (27) bestimmt ist.

4. Koaptor nach einem der Ansprüche 1 bis 3, dadurch gekennzeichnet, daß der Flügel (18 ; 37) von einem Stab (14 ; 36) gehalten ist, dessen Ende (15 ; 38) mit einer Einrichtung (19 ; 39) zur Steuerung seiner Schwenkbewegung versehen ist.

5. Koaptor nach einem der Ansprüche 1 bis 4, dadurch gekennzeichnet, daß der Flügel (18) die Form einer Scheibe aufweist, deren Durchmesser dem des Schaftes (8) ähnlich ist.

6. Koaptor nach einem der Ansprüche 1 bis 5, dadurch gekennzeichnet, daß der Flügel (37 ; 42) die Form eines Blattes hat.

7. Koaptor nach Anspruch 6, dadurch gekennzeichnet, daß der Flügel (42) die Form eines schraubenförmigen Blattes hat.

8. Koaptor nach einem der Ansprüche 1 bis 7, dadurch gekennzeichnet, daß der Schaft mit Längsrippen (11 ; 34) versehen ist, die zur Drehsicherung desselben dienen.

9. Koaptor nach einem der Ansprüche 1 bis 8, dadurch gekennzeichnet, daß der Schaft mit einer Längsnut (33) versehen ist, die zur Aufnahme eines Führungsdorns bestimmt ist.

FIG 1

FIG 2

FIG 3

FIG 4

FIG 5

FIG 6

FIG 8

FIG 9

FIG 9

FIG 10

FIG 11

FIG 12

FIG 13